(19) Europäisches Patentamt
European Patent Office
Office européen des brevets

(11) **EP 3 949 922 B1**

(12) **EUROPEAN PATENT SPECIFICATION**

(45) Date of publication and mention
of the grant of the patent:
**05.03.2025 Bulletin 2025/10**

(21) Application number: **20813132.6**

(22) Date of filing: **25.05.2020**

(51) International Patent Classification (IPC):
*A61F 13/49* (2006.01)    *A61F 13/494* (2006.01)
*A61F 13/496* (2006.01)

(52) Cooperative Patent Classification (CPC):
**A61F 13/49413**

(86) International application number:
**PCT/JP2020/020513**

(87) International publication number:
**WO 2020/241558 (03.12.2020 Gazette 2020/49)**

(54) **UNDERPANTS-TYPE ABSORBENT ARTICLE**

UNTERHOSENARTIGER SAUGFÄHIGER ARTIKEL

ARTICLE ABSORBANT DE TYPE SOUS-VÊTEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.05.2019 JP 2019103295**

(43) Date of publication of application:
**09.02.2022 Bulletin 2022/06**

(73) Proprietor: **Kao Corporation
Chuo-ku
Tokyo 103-8210 (JP)**

(72) Inventors:
• **MATSUDA, Kohei
Haga-gun, Tochigi 321-3497 (JP)**

• **KAWAGUCHI, Hiroko
Haga-gun, Tochigi 321-3497 (JP)**

(74) Representative: **Vossius & Partner
Patentanwälte Rechtsanwälte mbB
Siebertstraße 3
81675 München (DE)**

(56) References cited:
EP-A1- 1 880 700      WO-A1-2015/182441
WO-A1-2019/038956     WO-A1-2019/038956
JP-A- 2005 312 601    JP-A- 2005 312 601
JP-A- 2014 217 413    JP-A- 2014 217 413
JP-A- 2016 005 545    JP-A- 2016 005 545
JP-A- 2017 023 782    JP-A- 2017 023 782

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Technical Field

The present invention relates to a pull-on absorbent article, such as a disposable pull-on diaper.

Background Art

Absorbent articles such as disposable diapers and sanitary napkins may generally have a longitudinally extending leak-proof cuff disposed along laterally opposite sides thereof to provide protection against leakage of bodily discharges. JP 2017-023782A listed below teaches that, in the formation of a leak-proof cuff made of gathered nonwoven fabric which rises on contraction of an elastic member, the free edge portion of the upstanding cuff is embossed to be softened.

JP 2019-010348A proposes using, as a material forming leak-proof cuffs, nonwoven fabric characterized in terms of basis weight, bending stiffness and bending hysteresis measured with a bending tester, softness and smoothness measured with a tissue softness tester, and water pressure resistance measured with a water resistance tester with a view to improving the feel to the touch of the leak-proof cuffs.

WO 2019/038956 A1 discloses an absorbent article that has an absorbent main body equipped with an absorbent body provided with a narrow-width section, and also equipped with a leak-preventing wall having a fixed section and a rising section; a front waist section; and a rear waist section. Therein, one waist section overlaps the narrow-width section in the vertical direction on the non-skin side of the narrow-width section. In addition, the non-skin-side surface of the skin-side sheet section of the absorbent main body or the skin-side surface of the non-skin-side sheet section thereof is coated with an adhesive above the bottom end of the one waist section and to the outside of the narrow-width section. Furthermore, the fixed section overlaps the absorbent body from the skin side thereof, and with the intersection between the bottom end of the one waist section and a lateral end of the narrow-width section as a reference, the rising-start location of the rising section is positioned to the outside of said intersection in the left-right direction.

Summary of Invention

The present invention relates to a pull-on type absorbent article as defined in independent claim 1. Preferred aspects are defined in the dependent claims.

Brief Description of Drawings

[Fig. 1] Fig. 1 is a schematic perspective of an embodiment of the pull-on absorbent article of the present invention.
[Fig. 2] Fig. 2 is a schematic plan of the skin facing surface side (interior surface side) of the diaper shown in Fig. 1 in its flat-out, uncontracted configuration.
[Fig. 3] Fig. 3 is a schematic cross-section taken along line III-III in Fig. 2.
[Fig. 4] Fig. 4 is a schematic cross-section taken along line IV-IV in Fig. 2.
[Fig. 5] Fig. 5 is an enlarged view of an essential part of the plan shown in Fig. 2.
[Fig. 6] Fig. 6 is an enlarged schematic plan representing the surface of a fixed end portion of a leak-proof cuff.
[Fig. 7] Fig. 7 is an enlarged schematic plan illustrating the surface of a joined region inclusive of a fixed edge of a leak-proof cuff.

Description of Embodiments

A disposable pull-on diaper is put on by passing the wearer's legs through the leg openings. Because a leak-proof cuff is configured to upstand on contraction of the provided elastic member, it can easily catch the wearer's leg or the caregiver's hand and may sometimes be fitted onto the wearer with its inboard edge turned outboard. If the diaper is worn with the leak-proof cuff turned outboard, the cuff fails to serve its essential function to protect against leakage, allowing bodily discharge to leak. In addition, it is troublesome for a caregiver to check whether the leak-proof cuffs rise in position for every diaper change. JP 2017-023782 A and JP 2019-010348 A give no considerations to prevention of a leak-proof cuff from turning outward.

In the light of the above circumstances, the present invention relates to a pull-on absorbent article having leak-proof cuffs that feel comfortable to the touch and are less likely to be turned outboard while worn.

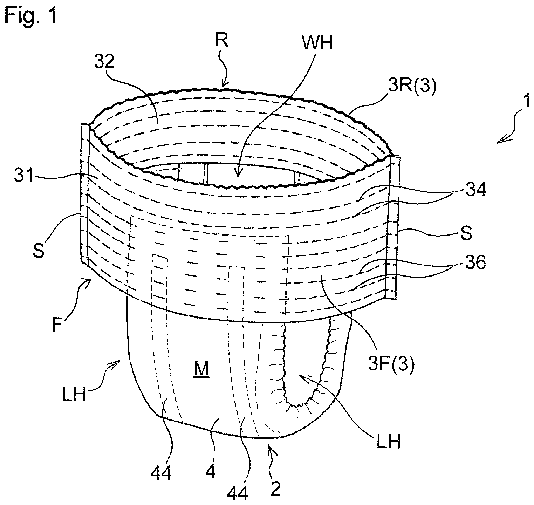

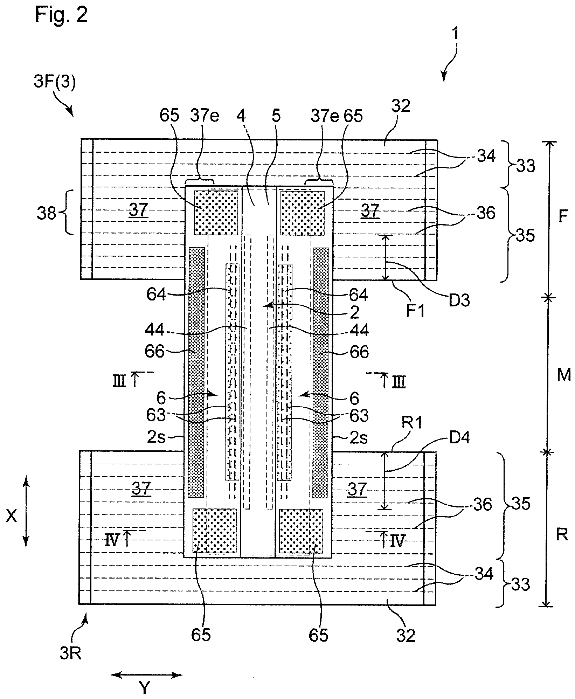

The present invention will be described on the basis of its preferred embodiments with reference to the accompanying drawings. Figs. 1 and 2 each illustrate a disposable pull-on diaper 1 as one embodiment of the present invention. As illustrated, the diaper 1 has a front portion F adapted to be applied to the front of a wearer, a rear portion R adapted to be applied to the back of a wearer, and a crotch portion M located between the front portion F and the rear portion R. The front portion F and the rear portion R are connected together to form a tubular, lower torso portion that is adapted to be worn about the lower torso of a wearer. The diaper 1 has a longitudinal direction X corresponding to the front-to-back direction of a wearer, i.e., the direction from the front portion F through the crotch portion M to the rear portion R, and a lateral direction Y perpendicular to the longitudinal direction X. The diaper 1 includes an absorbent assembly 2 and an outer cover 3 disposed on the non-skin facing surface side of the absorbent assembly 2.

As used herein, the term "skin facing surface" refers to the side of a disposable diaper or a member constituting the diaper (e.g., the absorbent assembly) facing the wearer's skin while worn, i.e., the side relatively closer to the wearer's skin. The term "non-skin facing surface" refers to the side of a disposable diaper or a member constituting the diaper facing away from the wearer's skin while worn, i.e., the side relatively farther from the wearer's skin. As used herein, the expression "while worn" means the state of a disposable diaper maintained in an ordinarily worn state, i.e., in the right position.

The front portion F and the rear portion R of the diaper 1 are joined together along their lateral side edges to form the outer cover 3 by a joining means, such as adhesive bonding, heat sealing, or ultrasonic sealing. As illustrated in Fig. 1, the diaper 1 is of pull-on type. The diaper 1 has a pair of side seams S, a waist opening WH for receiving the waist of a wearer, and a pair of leg openings LH for receiving the legs of the wearer.

The outer cover 3 includes a front panel 3F forming the front portion F and a rear panel 3R forming the rear portion R separately from the front panel 3F. The absorbent assembly 2 bridges the front panel 3F and the rear panel 3R separated at a prescribed spacing in the longitudinal direction X. As illustrated in Fig. 2, the panels 3F and 3R each have a rectangular shape longer in the lateral direction Y in a flat-out, uncontracted configuration (with the side seams S cut apart). As used herein, the expression "flat-out, uncontracted configuration (of the diaper 1)" means a state in which the diaper 1 is opened by cutting apart the side seams S and with every elastic member stretched out until the diaper is straightened up to its design dimension with any influences of elastic members eliminated.

The absorbent assembly 2 is, as illustrated in Fig. 2, rectangular in plan view and extends in the longitudinal direction X to straddle the front portion F and the rear portion R. The absorbent assembly 2 is joined to the lateral middle portion of the front panel 3F and the lateral middle portion of the rear panel 3R.

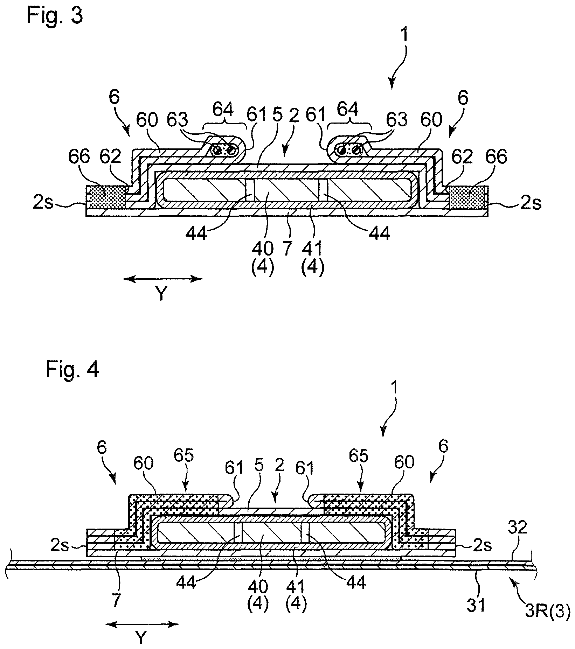

The absorbent assembly 2 is, as illustrated in Fig. 3, includes a liquid retentive absorbent member 4, a liquid permeable topsheet 5 providing the skin facing surface of the diaper 1, and a liquid impermeable or sparingly liquid impermeable leak-proof sheet 7 providing the non-skin facing surface of the diaper 1. The absorbent member 4 is longer in the longitudinal direction X of the diaper 1 similarly to the absorbent assembly 2. The topsheet 5 is provided on the skin facing surface side of the absorbent member 4, while the leak-proof sheet 7 on the non-skin facing surface side. The topsheet 5 and the leak-proof sheet 7 cover the entire upper and lower side, respectively, of the absorbent member 4, extend beyond the opposing, longitudinally extending lateral edges of the absorbent member 4, and joined together at their extensions by a known joining means, such as adhesive bonding. The absorbent member 4 is joined to both the topsheet 5 and the leak-proof sheet 7 by a known joining means, such as adhesive bonding.

As illustrated in Figs. 2 and 3, the absorbent member 4 includes an absorbent core 40 and a core wrap sheet 41 covering the absorbent core 40. The absorbent core 40 is formed of a fiber aggregate that may have an absorbent polymer dispersed therein. The core wrap sheet 41 is formed of tissue or water-permeable nonwoven fabric. The core wrap sheet 41 preferably covers the entire area of the skin facing surface side and non-skin facing surface side of the absorbent core 40.

As illustrated in Figs. 2 and 3, the absorbent assembly 2 has, on its skin facing surface, a leak-proof cuff 6 along either lateral side portion extending in the longitudinal direction X. In order for the leak-proof cuffs 6 to feel comfortable to the touch and slide easily on the wearer's skin while worn, it is preferable that the leak-proof cuffs 6 be formed of nonwoven fabric 60 that meets the following requirements of mean deviation of surface roughness (SMD) and mean deviation of coefficient of friction (MMD).

The nonwoven fabric 60 preferably has an SMD of 2.2 $\mu$m or less, more preferably 2.0 $\mu$m or less, even more preferably 1.8 $\mu$m or less. The closer to zero the SMD, the better, but an SMD of 0.5 $\mu$m would be sufficiently low to ensure sufficient softness and smoothness of the leak-proof cuff 6.

The nonwoven fabric 60 preferably has an MMD of 0.012 or less, more preferably 0.01 or less, even more preferably 0.008 or less. The closer to zero the MMD, the better, but an MMD of 0.005 would be sufficiently low to ensure sufficient softness and smoothness of the leak-proof cuff 6.

The SMD and MMD can be determined as follows using KESFB4-AUTO-A (trade name) from Kato Tech Co., Ltd. in accordance with the method described in Kawabata, Hideo, fuai hyouka no hyoujunka to kaiseki (Standardization and Analysis of Hand Evaluation), Japanese Hand Evaluation and Standardization Committee (HESC), The Textile Machinery Soc. of Japan, 2nd Ed. (Jul. 10, 1980).

Method for determining surface roughness mean deviation (SMD):

A sample measuring 20 cm by 20 cm is cut out of the nonwoven fabric forming the leak-proof cuff. When the nonwoven fabric being tested is short of that size, the size of the sample may be altered appropriately. The sample is clamped on a flat metal plate surface. A contact sensor is pressed onto the sample at a contact force of 9.8 cN (with an uncertainty of $\pm$0.49 cN), and the sample is moved horizontally at a constant speed of 0.1 cm/sec by 2 cm to be given a uniaxial tension of 7.3 cN/cm. The contact sensor is composed of a U-shaped single piano wire of 0.5 mm in diameter and 5 mm at the base. The contact sensor is urged onto the sample by a spring at a contact force of 9.8 cN. The spring had a spring constant of 24.5 cN/mm (with an uncertainty of $\pm$0.98 cN/mm) and a resonant frequency of at least 30 Hz out of contact with the sample surface. The mean deviation of surface roughness is represented in terms of SMD value. The measurement is taken in both the MD and CD to obtain $SMD_{MD}$ and $SMD_{CD}$, respectively, an average of which obtained by the following expression is taken as a mean deviation of surface roughness SMD. In the case where the nonwoven fabric sheet is too small to make specimens for MD and CD measurements, either $SMD_{MD}$ or $SMD_{CD}$ is adopted. The MD refers to the machine direction in the production of the nonwoven fabric or the longitudinal direction of the diaper, and the CD refers to a direction perpendicular to the MD.

$$\text{Mean deviation of surface roughness SMD} = \{(SMD_{MD}^2 + SMD_{CD}^2)/2\}^{1/2}$$

Method for determining mean deviation of friction coefficient (MMD):

A sample measuring 20 cm by 20 cm is cut out of the nonwoven fabric forming the leak-proof cuff. When the nonwoven fabric being tested is short of that size, the size of the sample may be altered appropriately. The sample is clamped on a flat metal plate surface. A contact sensor is pressed onto the sample at a contact force of 49 cN, and the sample is moved horizontally at a constant speed of 0.1 cm/sec by 2 cm to be given a uniaxial tension of 7.3 cN/cm. The contact sensor is composed of a set of 20 piano wires of 0.5 mm in diameter (each wire is the same as used in the measurement of surface roughness) arranged in parallel and U-shaped with a base width of 10 mm. The contact sensor is pressed onto the sample at a contact force of 49 cN using a weight. The mean deviation of friction coefficient is represented in terms of MMD value. The measurement is taken in both the MD and CD to obtain $MMD_{MD}$ and $MMD_{CD}$, respectively, an average of which obtained by the following expression is taken as a mean deviation of friction coefficient MMD. In the case where the nonwoven fabric sheet is too small to make specimens for MD and CD measurements, either $MMD_{MD}$ or $MMD_{CD}$ is adopted.

$$\text{Mean deviation of friction coefficient MMD} = \{(MMD_{MD}^2 + MMD_{CD}^2)/2\}^{1/2}$$

With the view to improving the smoothness against the skin of the leak-proof cuff 6, the nonwoven fabric 60 preferably has a coefficient of friction (MIU) of 0.25 or less, more preferably 0.18 or less. The closer to zero the MIU, the better, but the MIU of 0.15 would be sufficiently low to provide sufficient smoothness. The MIU can be determined using KESFB4-AUTO-A (trade name) from Kato Tech Co., Ltd. in accordance with the method described in the above-mentioned book, *fuai hyouka no hyoujunka to kaiseki* (Standardization and Analysis of Hand Evaluation).

Method for determining coefficient of friction (MIU):

A sample measuring 20 cm by 20 cm is cut out of the nonwoven fabric forming the leak-proof cuff. When the nonwoven fabric being tested is short of that size, the size of the sample may be altered appropriately. The sample is clamped on a flat metal plate surface. A contact sensor is the same as used in the surface roughness determination, i.e., composed of a set of 20 piano wires of 0.5 mm in diameter arranged in parallel and U-shaped with a base width of 10 mm. The contact sensor is pressed onto the sample at a contact force of 49 cN using a weight. The measurement is taken in both the MD and CD, and the average of the measurements is taken as an MIU. In the case where the sheet is too small to make specimens for MD and CD measurements, either one of them is used. The MD and CD are as defined above.

Each leak-proof cuff 6 is made of the nonwoven fabric 60 which meets the requirements of at least SMD and MMD out of the above-discussed three parameters: SMD, MMD, and MIU, that is, which has an SMD of 2.2 $\mu$m or less and an MMD of 0.012 or less. Each leak-proof cuff 6 is formed of two layers of the nonwoven fabric 60 facing to each other. The facing two layers of the nonwoven fabric may be of different types of nonwoven fabrics that meet the SMD and MMD requirements or may be of a single sheet of the nonwoven fabric that meet the SMD and MMD requirements folded in two. In the embodiment illustrated in Fig. 3 the leak-proof cuff 6 is formed of a single sheet of the nonwoven fabric 60 folded in two layers facing each other.

The leak-proof cuff 6 has opposing, longitudinally extending edges, one is a free edge 61 and the other is a fixed edge 62. The leak-proof cuff 6 includes at least one elastic member 63 adhesively fixed in its stretched state between the facing layers of the nonwoven fabric 60 to extend in the longitudinal direction X near along the free edge 61. Having the elastic member 63 fixed in its stretched state, the leak-proof cuff 6 is provided with an elasticized region 64 along the free edge 61. As illustrated in Fig. 3, the nonwoven fabric 60 forming the leak-proof cuff 6 is joined to the topsheet 5 in its side portion laterally opposite to the free edge 61. Specifically, the side portion of the nonwoven fabric 60 is joined to the part of the topsheet 5 that is located laterally outside of the side edge of the absorbent member 4 to form a joined region 66. The proximal edge of the joined region 66, which is closer to the free edge 61, corresponds to the fixed edge 62. The joined region 66 extends in the longitudinal direction X along the side edge 2s of the absorbent assembly 2 in at least the crotch portion M. Naturally, the fixed edge 62 also extends along the side edge 2s of the absorbent assembly 2.

As illustrated in Figs. 2 and 4, the leak-proof cuff 6 has its longitudinal end portions fixed to the topsheet 5 to form fixed end portions 65. Each fixed end portion 65 is longitudinally spaced from the longitudinal end of the elasticized region 64 in the front portion F and the rear portion R. Each fixed end portion 65 is formed by fixing the longitudinal end portion of the leak-proof cuff 6 inclusive of the free edge 61 to the topsheet 5 in each of the front portion F and the rear portion R. Therefore, the leak-proof cuff 6 is configured to upstand in the crotch portion M by the contraction of the elastic member 63 disposed in its stretched state. Bodily exudates, such as urine, is thus blocked from running laterally outward by the standing leak-proof cuff 6.

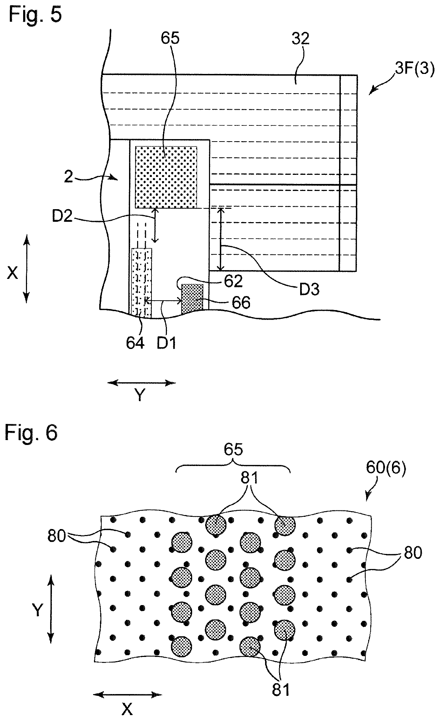

As illustrated in Fig. 5, in an uncontracted state of the diaper 1, the fixed edge 62 and the elasticized region 64 of the leak-proof cuff 6 are laterally spaced at a first distance D1. In order for the cuff 6 to exhibit sufficient softness, the first distance D1 is preferably at least 10 mm. To exhibit more sufficient softness, the first distance D1 is more preferably 15 mm or longer, even more preferably 20 mm or longer. The first distance D1 is the shortest distance between the most laterally proximal edge of the elasticized region 64 and the fixed edge 62. When the most proximal edge of the elasticized region 64 is indistinct, the first distance D1 means the shortest distance between the elastic member 63 and the fixed edge 62. When there is a plurality of elastic members 63 providing the elasticized portion 64 as in the diaper 1 shown in Fig. 3, the shortest distance from the elastic member 63 closest to the fixed edge 62 to the fixed edge 62 is taken as the first distance D1.

As illustrated in Fig. 5, the elasticized region 64 and the fixed end portion 65 are longitudinally spaced at a second distance D2. With a view to preventing the standing leak-proof cuff 6 from turning outward, the second distance D2 is preferably equal to or smaller than the first distance D1 in at least one of the front portion F and the rear portion R. To more certainly prevent the leak-proof cuff 6 from turning outward, the second distance D2 is preferably not longer than 15 mm, more preferably 10 mm or smaller. The second distance D2 is the shortest distance between a truly elasticized part of the elasticized region 64 and the fixed end portion 65. The term "truly elasticized part" of the elasticized region 64 means the truly, at least longitudinally extensible and contractible part of the elasticized region 64, being intended to exclude any deelasticized part having the elastic member 63 but exhibiting no extensibility nor contractibility and any deelasticized part having adhesive applied to fix the elastic member 63. The longitudinal end of the truly elasticized part can be identified, for example, as follows. The leak-proof cuff 6 is maintained in a longitudinally stretched out configuration and marked with oily ink at more than one position that is supposed to be the end of the elasticized region 64. The position of the mark that does not move upon the cuff's being relaxed or released from the tension is identified to be the end of the elasticized region 64.

In the diaper 1 of the embodiment, since the first distance D1 between the fixed edge 62 and the elasticized region 64 in the leak-proof cuff 6 is preferably 10 mm or longer, the cuff 6 is soft and comfortable to the touch. Generally speaking, as a leak-proof cuff increases in softness, the cuff is more apt to turn outboard, catching the wearer's leg or caregiver's hand when the wearer's leg is passed through the leg opening in putting on the diaper. With the diaper 1, in contrast, because the second distance D2 between the elasticized region 64 and the fixed end portion 65 is smaller than the first distance D1, the tensility of the elasticized region 64 is ready to be applied to the leak-proof cuff 6 so as to place back the outboard turned cuff 6. As a result of this, the leak preventive effect of the leak-proof cuff 6 improves.

Even if the leak-proof cuff 6 should be turned outboard during wear, such an inconvenience will be fixed easily because, for one thing, the cuff 6 is slippery against the wearer's skin and, for another, the cuff 6 easily rises from the vicinity of the fixed end portion 65 by the contraction of the elasticized region 64. The reason of this effect is that the leak-proof cuff 6 of the diaper 1 according to the present embodiment is formed of the nonwoven fabric 60 having an SMD of 2.2 μm or less and an MMD of 0.012 or less. As a result, the leak preventive function of the leak-proof cuff 6 improves. With a view to further enhancing the above effect, it is preferred that the second distance D2 be equal to or smaller than the first distance D1 in both the front portion F and the rear portion R.

In order to prevent the leak-proof cuff 6 from turning outward, the end of the truly elasticized part of the elasticized region 64 preferably overlaps the fixed end portion 65 in the thickness direction in at least one of the front portion F and the rear portion R. In that case, the second direction D2 = 0 mm. To produce the effect efficiently in preventing the leak-proof cuff 6 from turning outward, it is preferred that the end of the truly elasticized part of the elasticized region 64 overlap the fixed end portion 65 in the thickness direction in both the front portion F and the rear portion R.

The peel strength between the leak-proof cuff 6 and the topsheet 5 in the joined region 66 inclusive of the fixed edge 62 (hereinafter referred to as a second peel strength) is preferably equal to or greater than the peel strength between the leak-proof cuff 6 and the topsheet 5 in the fixed end portion 65 (hereinafter referred to as a first peel strength). With the second peel strength being equal to or greater than the first peel strength, even when the leak-proof cuff 6 catches the wearer's leg, the caregiver's hand, or the like in passing the wearer's leg through the leg opening LH in putting on the diaper 1, the leak-proof cuff 6 may start to separate from the topsheet 5 in the fixed end portion 65 earlier than in the joined region 66. As a result, the leak-proof cuff 6 being turned outboard is prevented from overburdening the skin while retaining the minimum capability to prevent leakage over the cuff 6 in the crotch portion M.

From the above-described viewpoint, the first peel strength of the fixed end portion 65 is preferably 0.2 N/20 mm or higher, more preferably 0.3 N/20 mm or higher, preferably 5.0 N/20 mm or lower, more preferably 2.0 N/20 mm or lower, preferably 0.2 to 5.0 N/20 mm, more preferably 0.3 to 2.0 N/20 mm. The second peel strength in the joined region 66 is preferably 1.3 N/20 mm or higher, more preferably 1.5 N/20 mm or higher, preferably 7.0 N/20 mm or lower, more preferably 6.0 N/20 mm or lower, preferably 1.3 to 7.0 N/20 mm, more preferably 1.5 to 6.0 N/20 mm.

The first and second peel strength are measured by the method below. The tensile tester used herein may be, for example, Tensilon RTC series available from A & D Co., Ltd.

Method for measuring first peel strength:

A rectangular specimen containing a fixed end portion 65 and measuring longer than 50 mm in the longitudinal direction X and 20 mm in the lateral direction Y is cut out from an unused absorbent article as stretched out until the gathers formed by the contraction of elastic members are smoothed. The cutting is made such that the fixed end portion 65 is located in one of the longitudinal end portions of the specimen. The nonwoven fabric 60 and the topsheet 5 are peeled apart from each other from the other longitudinal end, and the peeled portions are clamped in jaws of a tensile tester in a T-configuration at an initial jaw separation of 50 mm. The jaws are further separated at a rate of 300 mm/min to record the maximum strength. The measurement is taken in triplicate, and the average maximum strength is defined to be a first peel strength (N/20 mm). When the specimen is shorter than 20 mm in width, the average maximum strength as measured is converted to a value in N/20 mm width.

Method for measuring second peel strength:

A rectangular specimen containing the joined region 66 and measuring longer than 50 mm in the lateral direction Y and 20 mm in the longitudinal direction X is cut out from an unused absorbent article. The nonwoven fabric 60 and the member joined thereto (e.g., the topsheet 5) are peeled apart from each other from one longitudinal end of the specimen, and the peeled portions are clamped in jaws of a tensile tester in a T-configuration at an initial jaw separation of 50 mm. The jaws are further separated at a rate of 300 mm/min to record the maximum strength. In testing the joined region 66 located on the wearer's right side, peeling is from left to right of the pull-on absorbent article. In testing the joined region 66 located on the wearer's left side, peeling is from right to left of the article. The measurement is taken in triplicate, and the average maximum strength is defined to be a second peel strength (N/20 mm).

With the view to preventing the leak-proof cuff 6 from turning outward, it is preferred that, as depicted in Fig. 2, the distance D3 between the laterally extending proximal edge F1 of the front panel 3F and the fixed end portion 65 of the front portion F be smaller than the distance D4 between the laterally extending proximal edge R1 of the rear panel 3R and the fixed end portion 65 of the rear portion R. The difference between the distances D4 and D3 is preferably at least 10 mm, more preferably 30 mm or greater, preferably 90 mm or smaller, more preferably 70 mm or smaller.

The distance D3 is preferably 5 mm or greater, more preferably 10 mm or greater, preferably 50 mm or smaller, more preferably 40 mm or smaller. The distance D4 is preferably 30 mm or greater, more preferably 50 mm or greater, preferably 100 mm or smaller, more preferably 90 mm or smaller.

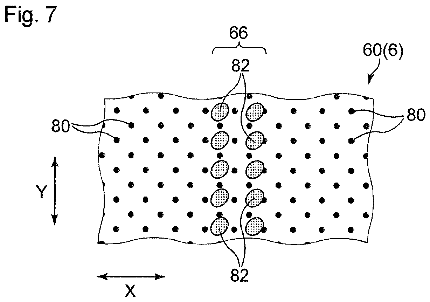

As shown in Figs. 6 and 7, it is preferred to use, as the nonwoven fabric 60, nonwoven fabric having depressions 80, where the constituent fibers are united, formed by embossing in a discrete pattern in plan view. In view of softness, the nonwoven fabric preferably contains as a main component of constituent fibers a propylene random copolymer composed of propylene and an $\alpha$-olefin other than propylene. Examples of suitable nonwoven fabrics include spun-bonded, melt-blown, and spun-bonded/melt-blown laminated nonwoven fabric.

With the view to preventing the leak-proof cuff 6 from turning outward, the total area ratio of the depressions 80 formed on one surface of the nonwoven fabric 60 is preferably 18% or less, more preferably 15% or less, preferably 8% or more, more preferably 10% or more, preferably 8% to 18%, and more preferably 10% to 15%.

The individual depressions 80 preferably have an area of 0.2 mm$^2$ or more, more preferably 0.3 mm$^2$ or more, preferably 0.5 mm$^2$ or less, more preferably 0.4 mm$^2$ or less.

The distance between one depression 80 and a closest depression 80 is preferably 0.8 mm or greater, more preferably 1.0 mm or greater, preferably 3.0 mm or smaller, more preferably 2.0 mm or smaller.

The fixed end portion 65 can be formed by a known joining method, such as heat embossing, high-frequency sealing, ultrasonic sealing, or hot-melt adhesive bonding. As illustrated in Fig. 6, the fixed end portion 65 of the diaper 1 has a plurality of fusion bonds 81 where the nonwoven fabric 60 and the topsheet 5 are partially fusion bonded together. With the view to preventing the leak-proof cuff 6 from turning outward, it is preferred that some of the fusion bonds 81 each overlap the depression 80. The ratio of the fusion bonds 81 overlapping the depression(s) 80 to all the fusion bonds 81 in the fixed end portion 65 is preferably 50% or higher, more preferably 60% or higher, preferably 90% or lower, more preferably 80% or lower. As used herein, the term "overlapping" is intended to include the relation in which the outline of a depression 80 and that of a fusion bond 81 are in contact with each other.

With the view to preventing the leak-proof cuff 6 from turning outward, the ratio of the total area of the fusion bonds 81 to the area of the fixed end portion 65 is preferably at least 5%, more preferably 10% or more, preferably 30% or less, more preferably 20% or less. The area of the fixed end portion 65 is calculated by multiplying its greatest dimensions in the longitudinal and lateral directions X and Y.

With the view to preventing the leak-proof cuff 6 from turning outward, the fixed end portion 65 preferably has an area of at least 300 mm$^2$, more preferably 800 mm$^2$ or more, preferably 1000 mm$^2$ or less, more preferably 900 mm$^2$ or less.

The fusion bonds 81 may be arranged in, for example, a latticed, staggered, honeycomb or any other patterns. The arrangement pattern illustrated in Fig. 6 is a staggered pattern that is effective in preventing the leak-proof cuff 6 from peeling off the topsheet 5. Specifically, the fusion bonds 81 form lines along the longitudinal direction X such that the fusion bonds 81 in a line are each located between longitudinally adjacent fusion bonds 81 in a laterally adjacent line.

The individual fusion bonds 81 may have any plan-view shape, such as circular, elongated circular, rectangular, triangular, and the like. From the viewpoint of the feel to the touch, the fusion bonds 81 in Fig. 6 are circular in plan view.

In order to allow the fusion bonds 81 to overlap the depression 80 easily, the individual fusion bonds 81 preferably have an area of at least 0.5 mm$^2$, more preferably 2.0 mm$^2$ or more, preferably 10.0 mm$^2$ or less, more preferably 5.0 mm$^2$ or less. From the standpoint of the balance between the feel to the touch of the leak-proof cuff 6 and the peel strength from the topsheet 5, it is preferred that the more laterally proximal the fusion bond 81 is, the larger the area. For easy overlapping between the fusion bonds 81 and the depressions 80, the distance between one fusion bond 81 and a closest fusion bond 81 is preferably 3 mm or greater, more preferably 5 mm or greater, preferably 10 mm or smaller, more preferably 8 mm or smaller.

The joined region 66 can be formed by a known joining method, such as heat embossing, high-frequency sealing, ultrasonic sealing, or hot-melt adhesive bonding, similarly to the fixed end portion 65. As illustrated in Fig. 7, the joined region 66 of the diaper 1 has a plurality of second fusion bonds 82 formed by heat embossing, high-frequency sealing, or ultrasonic sealing, where the nonwoven fabric 60 and the topsheet 5 are partially fusion bonded together. With a view to preventing leakage from the leak-proof cuff 6 in the crotch portion M, the area of the individual second fusion bonds 82 is preferably larger than that of the individual fusion bonds 81. The area of the individual second fusion bonds 82 is preferably at least 2 mm$^2$, more preferably 4 mm$^2$ or larger, preferably 20 mm$^2$ or smaller, more preferably 15 mm$^2$ or smaller. With the same view, the distance between one second fusion bond 82 and a closest second fusion bond 82 is preferably 0.5 mm or greater, more preferably 1 mm or greater, preferably 5 mm or smaller, more preferably 3 mm or smaller.

Method for measuring areas of depressions 80 and fusion bonds 81 and 82:

A leak-proof cuff 6 is cut apart from the diaper 1, and a 10 mm by 10 mm specimen is cut out from the region having the fusion bonds 81 or 82. The specimen is subjected to image analysis using, e.g., VHX-1000 from Keyence to measure the area of every fusion bond except fusion bonds with part cut away. The average of all the measurements is defined to be the area of the individual fusion bonds. The area of the individual depressions 80 is measured in the same manner. That is, a 10 mm by 10 mm specimen is cut out from the region having the depressions 80 of the nonwoven fabric 60 forming the leak-proof cuff 6. The specimen is analyzed using an image analyzer to measure the area of the individual depressions 80. The ratio of the total area of the fusion bonds 81 to the area of the fixed end portion 65 is calculated in terms of the total area of the fusion bonds 81 per unit area (10 mm by 10 mm = 100 mm$^2$) of the fixed end portion 65. The measurement is taken in triplicate, and the average of the three measurements is defined to be the ratio of the total area of the fusion bonds to the area of the fixed end portion. In the determination of the fusion bond area ratio, the area measurement is taken of all the fused bonds inclusive of those with part cut away. The ratio of the total area of the depressions 80 to the entire area of one surface of the nonwoven fabric 60 is calculated in terms of the total area of the depressions 80 per unit area (10 mm by 10 mm = 100 mm$^2$) of the specimen. At least three specimens are prepared from the same nonwoven fabric, and the average of the measurements is defined to be the ratio of the total area of the depressions 80 to the entire area of one surface of the nonwoven fabric.

The individual second fusion bonds 82 may have any plan-view shape, such as circular, elongated circular, rectangular, triangular, and the like. From the viewpoint of prevention of the leak-proof cuff 6 turning outward, the second fusion bonds 82 in Fig. 7 are elongated circular in plan view.

It is preferred that the joined region 66 have a smaller lateral dimension than the fixed end portion 65 as illustrated in Fig. 5 so that, when, in putting on the diaper 1, the leak-proof cuff 6 catches the leg of a wearer or the hand of a caregiver, the leak-proof cuff 6 may be easily allowed to right itself around the joined region 66 by the stress applied to the cuff 6. The term "lateral dimension of the joined region 66" means the greatest dimension in the lateral direction Y, and so does the term "lateral dimension of the fixed end portion 65".

With the view to preventing the leak-proof cuff 6 from turning outward, the joined region 66 is preferably longer than the longitudinally extending, truly elasticized part of the elasticized region 64 in the longitudinal direction X.

As illustrated in Fig. 4, the front panel 3F and the rear panel 3R, which form the outer cover 3, each include an outer sheet 31 and an inner sheet 32. While worn, the outer sheet 31 is located farther from the wearer's body to form the exterior surface, i.e., the non-skin facing surface of the diaper 1 while the inner sheet 32 is located closer to the wearer's body to form the interior surface, i.e., the skin facing surface of the diaper 1. The outer and inner sheets 31 and 32 are joined together at prescribed regions into a laminate sheet via a joining means, such as adhesive. The outer cover 3 includes a waist region along the edge of the waist opening WH and a lower torso region. The lower torso region is a region from a position 20 mm downward from the edge of the waist opening WH to the upper end of the leg opening LH. As shown in Fig. 2, the waist region and the lower torso region have waist elastic members 34 and lower torso elastic members 36, respectively, between the outer sheet 31 and the inner sheet 32 to form an elasticized waist region 33 and an elasticized lower torso region 35, respectively. The elastic members 34 and 36 are each fixed in their stretched state between the sheets 31 and 32 by any joining means, such as hot-melt adhesive bonding.

As illustrated in Fig. 2, the elasticized waist region 33 is located closer to the waist opening than the longitudinal end of the absorbent member 4 and extends along the circumferential direction of the waist region along the edge of the waist opening WH. The elasticized waist region 33 are laterally elasticized by the waist elastic members 34 fixed in their stretched state.

As illustrated in Fig. 2, the elasticized lower torso region 35 includes a pair of elasticized side regions 37 located laterally outside of the absorbent assembly 2. The elasticized side regions 37 are laterally spaced away from each other. Each elasticized side region 37 extends laterally at least outward from the fixed end portion 65. Each elasticized side region 37 is extensible and contractible in the lateral direction Y by the lower torso elastic members 36 fixed in their stretched state. Each elasticized side region 37 includes an elasticized fixed end region 38. The elasticized fixed end region 38 contains the fixed end portion 65 and extends laterally outward from the fixed end portion 65. In order to allow the leak-proof cuff 6 to easily rise from near the fixed end portion 65 and to prevent the leak-proof cuff 6 from turning outward, the elasticized fixed end region 38 of the elasticized side region 37 has a higher lateral extensional stress than the elasticized waist region 33. The extensional stress can be measured as follows.

Method for measuring extensional stress:

Specimens are prepared by laterally cutting a diaper in a flat-out configuration. The elasticized waist region and the elasticized fixed end region 38 are cut out from each of the front portion F and the rear portion R. The elasticized waist region is a region from the edge of the waist opening WH to the longitudinal end of the absorbent member 4 in which the elastic members 34 are disposed, and the elasticized fixed end region 38 is the part of the elasticized side region 37 that is located at the same position as the fixed end portion 65 in the longitudinal direction X and has the elastic members 36 disposed in the lateral direction Y. The diaper is cut such that the specimen of the elasticized fixed end region 38 includes not only the outer cover 3 but all the other members present in the thickness direction (e.g., the absorbent assembly 2). The longitudinal ends of each specimen are clamped in jaws of Tensilon tensile tester (RTC-1210A from Orientec) at positions just inside the opposing side seams S and pulled at a rate of 300 mm/min. The inner lateral dimension (the distance between the pair of side seams S) of the outer cover 3 in its uncontracted state (i.e., in a smoothed-out configuration with no elastic member disposed), e.g., 350 mm is taken as 100. The specimen is once extended up to 80 (corresponding to, e.g., 280 mm) and then retracted to 71 (corresponding to, e.g., 250 mm). The tensile load (cN) at 71 retraction is defined to be the contraction force. The reason of adopting the stress at 71 retraction (relative to the inner dimension of the outer cover 3 taken as 100) is because the ratio of the abdominal circumference of children, the principal intended users of the diaper 1, is about 71% of the inner dimension of the diaper. As used herein, the term "abdominal circumference" refers to an average of the measurement in a standing position and that in a sitting position, taking into consideration the change in abdominal circumference of a child with change in posture.

The ratio of the extensional stress of the elasticized fixed end region 38 to that of the elasticized waist region 33 (extensional stress of the elasticized fixed end region 38 / extensional stress of the elasticized waist region 33) is preferably 1.04 or higher, more preferably 1.05 or higher, preferably 3.0 or lower, more preferably 2.5 or lower.

The extensional stress of the elasticized waist region 33 is preferably 0.6 cN/mm or higher, more preferably 0.8 cN/mm or higher, preferably 5.5 cN/mm or lower, more preferably 4.5 cN/mm or lower. The extensional stress of the elasticized side region 37 is preferably 0.8 cN/mm or higher, more preferably 1.0 cN/mm or higher, preferably 7.0 cN/mm or lower, more preferably 6.0 cN/mm or lower.

With a view to achieving a close conforming fit to the wearer's body and helping the leak-proof cuff 6 to rise from near the fixed end portion 65, it is preferred that each elasticized fixed end region 38 overlaps the fixed end portion 65 in its laterally proximal end portion 37e in the thickness direction as illustrated in Fig. 2.

The absorbent core 40 has a single layer structure as illustrated in Fig. 3. The absorbent core 40 may or may not have a flexure region in the inside in the lateral direction. The flexure region may be a slit formed through the thickness of the absorbent core 40 or a low basis weight region having a lower basis weight than the surroundings. The flexure region serves as a hinge helping the opposite lateral side portions of the absorbent core 40 to bend upward, thereby preventing bodily waste from running laterally outside. The absorbent core 40 shown in Figs. 3 and 4 has a pair of slits 44 extending in the longitudinal direction X as a flexure region. The slits 44 are formed laterally inward of the side edges of the absorbent member 4. The low basis weight region which may be provided in place of the slit 44 preferably has a basis weight of 10 to 400 g/mm$^2$, more preferably 100 to 300 g/mm$^2$. The absorbent core 40 may have a multi-layer structure.

To facilitate the upward bending of the side portions of the absorbent member 4 and to prevent the leak-proof cuff 6 from turning outward, the slit 44 preferably extends in the longitudinal direction X in parallel with the joined region 66. For the same purpose, the fixed end portion 65 is preferably located laterally outside of the slit. In providing a low basis weight region instead of the slit 44, the above-described preferred positional relationship equally applies.

Materials making constituent members of the diaper 1 are not particularly limited and may be selected from those commonly used in the art. For example, the topsheet 5 may be formed of various types of nonwoven fabric or perforated film. The leak-proof sheet 7 may be formed of sparingly liquid-permeable resin film or spun-bonded/melt-blown/spun-bonded laminated nonwoven fabric. The elastic members may be rubber threads. The outer sheet 31 and the inner sheet 32 may be formed of various types of nonwoven fabric.

The present invention has been described on the basis of its preferred embodiments, but it should be understood that the present invention is not deemed to be limited thereto, and various changes and modifications can be made therein. For example, while the outer cover of the embodiment illustrated in Fig. 1 is divided into the front and the rear panel, the outer cover may be continuous from the front portion A to the rear portion R through the crotch section M. The pull-on absorbent article of the present invention may be a panty-type sanitary napkin or mild incontinence underwear as well as a disposable pull-on diaper.

While in Fig. 3 the joined region 66 formed of the leak-proof cuff 6 and the topsheet 5 is located distally to the side edge of the absorbent member 4, the joined region 66 may be formed by joining the part of the topsheet that wraps around to the non-skin facing surface side of the absorbent member 4 and the nonwoven fabric 60 of the leak-proof cuff 6. Furthermore, the joined region 66 may be formed by joining the nonwoven fabric 60 of the leak-proof cuff 6 not to the topsheet 5 but another member constituting the absorbent assembly 2, such as the leak-proof sheet 7.

Industrial Applicability

The leak-proof cuffs of the pull-on absorbent article of the invention have a good feel to the touch and are yet prevented from turning outward and thereby provide improved protection against leakage.

**Claims**

1. A pull-on absorbent article having a longitudinal direction (X) corresponding to the front-to-back direction of a wearer and a lateral direction (Y) perpendicular to the longitudinal direction (X), having a front portion (F) adapted to be worn about a wearer's front, a rear portion (R) adapted to be worn about a wearer's back, and a crotch portion (M) intermediate between the front and rear portions, and having a waist opening (WH) and a pair of leg opening (LH),

   the pull-on absorbent article comprising an absorbent assembly (2) comprising a liquid permeable topsheet (5), an absorbent member (4) and a leak-proof sheet (7) and a leak-proof cuff (6) extending in the longitudinal direction (X) along each lateral side of the absorbent assembly (2),
   each leak-proof cuff (6) being formed of nonwoven fabric (60) and having a fixed edge (62) along one of its lateral side edges and a free edge (61) on the other side,
   each leak-proof cuff (6) having opposite longitudinal ends, one in the front portion (F) and the other in the rear portion (R), and having a fixed end portion (65) fixed to the topsheet (5) near each longitudinal end,
   each leak-proof cuff (6) having an elasticized region (64) in which an elastic member (63) is fixed along near the free edge (61),
   the fixed edge (62) and the elasticized region (64) of each leak-proof cuff (6) being laterally spaced at a first distance (D1) of 10 mm or longer, and the elasticized region (64) and the fixed end portion (65) of each leak-proof cuff (6) being longitudinally spaced at a second distance (D2), the second distance (D2) being equal to or smaller than the first distance (D1) in at least one of the front portion (F) and the rear portion (R), and
   the nonwoven fabric (60) having a mean deviation of surface roughness (SMD) of 2.2 $\mu$m or less and a mean deviation of coefficient of friction (MMD) of 0.012 or less, the mean deviation of surface roughness and the mean deviation of coefficient of friction being measured according to the method described in the description,

wherein the absorbent assembly (2) comprises the absorbent member (4),

at least one of the front portion (F) and the rear portion (R) comprises a laterally extending elasticized waist region (33) located closer to the waist opening (WH) than the longitudinal end of the absorbent member (4) and a pair of laterally extending elasticized side regions (37) each located laterally outside of the absorbent assembly (2), each elasticized side region (37) comprises an elasticized fixed end region (38), the elasticized fixed end region (38) containing the fixed end portion (65) and extending laterally outward from the fixed end portion (65), and the elasticized fixed end region (38) has a higher extensional stress than the elasticized waist region (33).

2. The pull-on absorbent article according to claim 1, wherein the second distance (D2) is equal to or smaller than the first distance (D1) in both the front and rear portions.

3. The pull-on absorbent article according to claim 1 or 2, wherein the fixed end portion (65) has a first peel strength of 0.2 to 5.0 N/20 mm between the nonwoven fabric (60) and the topsheet (5), and a joined region (66) inclusive of the fixed edge (62) has a peel strength between the nonwoven fabric (60) and a member joined thereto equal to or greater than the first peel strength, the peel strength being measured according to the method described in the description.

4. The pull-on absorbent article according to claim 3, wherein the peel strength of the joined region (66) inclusive of the fixed edge (62) is 1.3 N/20 mm or greater.

5. The pull-on absorbent article according to claim 3 or 4, wherein the joined region (66) inclusive of the fixed edge (62) has a smaller lateral dimension than the fixed end portion (65).

6. The pull-on absorbent article according to any one of claims 1 to 5, wherein the first distance (D1) is 15 mm or longer.

7. The pull-on absorbent article according to any one of claims 1 to 6, wherein the second distance (D2) is 15 mm or smaller.

8. The pull-on absorbent article according to any one of claims 1 to 7, wherein the fixed end portion (65) has a plurality of fusion bonds (81) where the nonwoven fabric (60) and the topsheet (5) are fusion bonded together, and
the ratio of the total area of the fusion bonds (81) to the area of the fixed end portion (65) is at least 5%.

9. The pull-on absorbent article according to any one of claims 1 to 8, wherein further comprising an outer cover (3) to which the absorbent assembly (2) is fixed, the outer cover (3) comprising a front panel (3F) in the front portion (F) and a rear panel (3R) in the rear portion (R).

10. The pull-on absorbent article according to any one of claims 1 to 9, wherein at least one of the front portion (F) and the rear portion (R) includes: a laterally extending elasticized waist region (33) located closer to the waist opening (WH) than the end of the absorbent member (4) and extending along a circumferential direction of the waist region (33); and a pair of laterally extending elasticized side regions (37) each located laterally outside of the absorbent assembly (2),

each elasticized side region (37) comprises an elasticized fixed end region (38) containing the fixed end portion (65) and extending laterally outward from the fixed end portion (65),
the elasticized fixed end region (38) has a higher extensional stress than the elasticized waist region (33), the extensional stress being measured according to the method described in the description.

11. The pull-on absorbent article according to claim 10, wherein each elasticized fixed end region overlaps the fixed end portion in its laterally proximal end portion in the thickness direction

12. The pull-on absorbent article according to any one of claims 1 to 11, wherein each elasticized fixed end region (38) overlaps the fixed end portion (65) in its laterally proximal end portion in the thickness direction.

13. The pull-on absorbent article according to any one of claims 1 to 12, wherein the absorbent assembly (2) comprises an absorbent member (4), and
the absorbent member (4) has a slit (44) or a low basis weight region in its opposite side portions, the slit (44) or low basis weight region extending longitudinally in parallel with a joined region (66) inclusive of the fixed edge (62).

14. The pull-on absorbent article according to claim 13, wherein the fixed end portion (65) is located laterally outside of the slit (44) or the low basis weight region.

**Patentansprüche**

1. Saugfähiger Artikel vom Höschen-Typ, der eine Längsrichtung (X), die der Vorderseite-zu-Rückseite-Richtung eines Trägers entspricht, und eine Querrichtung (Y) senkrecht zur Längsrichtung (X) hat, einen vorderen Abschnitt (F), der so angepasst ist, dass er um eine Vorderseite eines Trägers getragen werden kann, einen hinteren Abschnitt (R), der so angepasst ist, dass er um eine Rückseite eines Trägers getragen werden kann, und einen Schrittabschnitt (M) zwischen dem vorderen und dem hinteren Abschnitt hat, und eine Taillenöffnung (WH) und ein Paar Beinöffnungen (LH) hat,

   wobei der saugfähige Artikel vom Höschen-Typ eine saugfähige Anordnung (2) aufweist, die eine flüssigkeitsdurchlässige Oberschicht (5), ein saugfähiges Element (4) und eine auslaufsichere Schicht (7) und ein auslaufsicheres Bündchen (6) aufweist, das sich in der Längsrichtung (X) entlang jeder seitlichen Seite der saugfähigen Anordnung (2) erstreckt,
   wobei jedes auslaufsichere Bündchen (6) aus Vliesstoff (60) gebildet ist und einen festen Rand (62) entlang einer seiner seitlichen Seitenkanten und einen freien Rand (61) auf der anderen Seite aufweist,
   wobei jedes auslaufsichere Bündchen (6) gegenüberliegende Längsenden hat, eines im vorderen Abschnitt (F) und das andere im hinteren Abschnitt (R), und einen festen Endabschnitt (65) hat, der an der Oberschicht (5) nahe jedem Längsende befestigt ist,
   wobei jedes auslaufsichere Bündchen (6) einen elastischen Bereich (64) hat, in dem ein elastisches Element (63) in der Nähe und entlang des freien Randes (61) befestigt ist,
   wobei der feste Rand (62) und der elastische Bereich (64) jedes auslaufsicheren Bündchens (6) seitlich in einem ersten Abstand (D1) von 10 mm oder mehr beabstandet sind, und der elastische Bereich (64) und der feste Endabschnitt (65) jedes auslaufsicheren Bündchens (6) in Längsrichtung in einem zweiten Abstand (D2) beabstandet sind, wobei der zweite Abstand (D2) gleich oder kleiner als der erste Abstand (D1) in dem vorderen Abschnitt (F) und/oder dem hinteren Abschnitt (R) ist, und
   wobei der Vliesstoff (60) eine mittlere Abweichung der Oberflächenrauhigkeit (SMD) von 2,2 $\mu$m oder weniger und eine mittlere Abweichung des Reibungskoeffizienten (MMD) von 0,012 oder weniger aufweist, wobei die mittlere Abweichung der Oberflächenrauhigkeit und die mittlere Abweichung des Reibungskoeffizienten gemäß dem in der Beschreibung beschriebenen Verfahren gemessen werden,
   wobei die saugfähige Anordnung (2) das saugfähige Element (4) aufweist,
   wobei der vordere Abschnitt (F) und/oder der hintere Abschnitt (R) einen sich seitlich erstreckenden elastischen Taillenbereich (33), der näher an der Taillenöffnung (WH) als das Längsende des saugfähigen Elements (4) angeordnet ist, und ein Paar sich seitlich erstreckende elastische Seitenbereiche (37) aufweist, die jeweils seitlich außerhalb der saugfähigen Anordnung (2) angeordnet sind,
   wobei jeder elastische Seitenbereich (37) einen elastischen festen Endbereich (38) aufweist, wobei der elastische feste Endbereich (38) den festen Endabschnitt (65) enthält und sich von dem festen Endabschnitt (65) seitlich nach außen erstreckt, und
   wobei der elastische feste Endbereich (38) eine höhere Dehnungsspannung aufweist als der elastische Taillenbereich (33).

2. Saugfähiger Artikel vom Höschen-Typ nach Anspruch 1, wobei der zweite Abstand (D2) sowohl im vorderen als auch im hinteren Abschnitt gleich oder kleiner als der erste Abstand (D1) ist.

3. Saugfähiger Artikel vom Höschen-Typ nach Anspruch 1 oder 2, wobei der feste Endabschnitt (65) eine erste Schälfestigkeit von 0,2 bis 5,0 N/20 mm zwischen dem Vliesstoff (60) und der Oberschicht (5) aufweist und ein verbundener Bereich (66) einschließlich des festen Randes (62) eine Schälfestigkeit zwischen dem Vliesstoff (60) und einem damit verbundenen Element aufweist, die gleich oder größer ist als die erste Schälfestigkeit, wobei die Schälfestigkeit gemäß dem in der Beschreibung beschriebenen Verfahren gemessen wird.

4. Saugfähiger Artikel vom Höschen-Typ nach Anspruch 3, wobei die Schälfestigkeit des verbundenen Bereichs (66) einschließlich des festen Randes (62) 1,3 N/20 mm oder mehr beträgt.

5. Saugfähiger Artikel vom Höschen-Typ nach Anspruch 3 oder 4, wobei der verbundene Bereich (66) einschließlich des festen Randes (62) eine kleinere seitliche Abmessung hat als der feste Endabschnitt (65).

**6.** Saugfähiger Artikel vom Höschen-Typ nach einem der Ansprüche 1 bis 5, wobei der erste Abstand (D1) 15 mm oder länger ist.

**7.** Saugfähiger Artikel vom Höschen-Typ nach einem der Ansprüche 1 bis 6, wobei der zweite Abstand (D2) 15 mm oder weniger ist.

**8.** Saugfähiger Artikel vom Höschen-Typ nach einem der Ansprüche 1 bis 7, wobei der feste Endabschnitt (65) eine Vielzahl von Schmelzverbindungen (81) aufweist, an denen der Vliesstoff (60) und die Oberschicht (5) miteinander verschmolzen sind, und
das Verhältnis der Gesamtfläche der Schmelzverbindungen (81) zur Fläche des festen Endabschnitts (65) mindestens 5% beträgt.

**9.** Saugfähiger Artikel vom Höschen-Typ nach einem der Ansprüche 1 bis 8, der ferner eine äußere Abdeckung (3) aufweist, an der die saugfähige Anordnung (2) befestigt ist, wobei die äußere Abdeckung (3) ein vorderes Element (3F) im vorderen Abschnitt (F) und ein hinteres Element (3R) im hinteren Abschnitt (R) aufweist.

**10.** Saugfähiger Artikel vom Höschen-Typ nach einem der Ansprüche 1 bis 9, wobei der vordere Abschnitt (F) und/oder der hintere Abschnitt (R) aufweist: einen sich seitlich erstreckenden elastischen Taillenbereich (33), der näher an der Taillenöffnung (WH) als das Ende des saugfähigen Elements (4) angeordnet ist und sich entlang einer Umfangsrichtung des Taillenbereichs (33) erstreckt; und ein Paar sich seitlich erstreckende elastische Seitenbereiche (37), die jeweils seitlich außerhalb der saugfähigen Anordnung (2) angeordnet sind,

wobei jeder elastische Seitenbereich (37) einen elastischen festen Endbereich (38) aufweist, der den festen Endabschnitt (65) enthält und sich von dem festen Endabschnitt (65) seitlich nach außen erstreckt,
wobei der elastische feste Endbereich (38) eine höhere Dehnungsspannung aufweist als der elastische Taillenbereich (33), wobei die Dehnungsspannung gemäß dem in der Beschreibung beschriebenen Verfahren gemessen wird.

**11.** Saugfähiger Artikel vom Höschen-Typ nach Anspruch 10, wobei jeder elastische feste Endbereich den festen Endabschnitt in seinem seitlich proximalen Endabschnitt in der Dickenrichtung überlappt.

**12.** Saugfähiger Artikel vom Höschen-Typ nach einem der Ansprüche 1 bis 11, wobei jeder elastische feste Endbereich (38) den festen Endabschnitt (65) in seinem seitlich proximalen Endabschnitt in der Dickenrichtung überlappt.

**13.** Saugfähiger Artikel vom Höschen-Typ nach einem der Ansprüche 1 bis 12, wobei die saugfähige Anordnung (2) ein saugfähiges Element (4) aufweist, und
das saugfähige Element (4) einen Schlitz (44) oder einen Bereich mit niedrigem Flächengewicht in seinen gegenüberliegenden Seitenabschnitten aufweist, wobei sich der Schlitz (44) oder der Bereich mit niedrigem Flächengewicht in Längsrichtung parallel zu einem verbundenen Bereich (66) einschließlich des festen Randes (62) erstreckt.

**14.** Saugfähiger Artikel vom Höschen-Typ nach Anspruch 13, wobei sich der feste Endabschnitt (65) seitlich außerhalb des Schlitzes (44) oder des Bereichs mit geringem Flächengewicht befindet.

## Revendications

**1.** Article absorbant à enfiler ayant une direction longitudinale (X) correspondant à la direction avant/arrière d'un porteur, et une direction latérale (Y) perpendiculaire à la direction longitudinale (X), ayant une portion avant (F) adaptée pour être portée autour du devant d'un porteur, une portion arrière (R) adaptée pour être portée autour du derrière d'un porteur, et une portion d'entrejambe (M) intermédiaire entre les portions avant et arrière, et ayant une ouverture de taille (WH) et une paire d'ouvertures de jambe (LH),

l'article absorbant à enfiler comprenant un assemblage absorbant (2) comprenant une feuille supérieure perméable aux liquides (5), un élément absorbant (4) et une feuille anti-fuite (7) et un revers anti-fuite (6) s'étendant dans la direction longitudinale (X) le long de chaque côté latéral de l'assemblage absorbant (2), chaque revers anti-fuite (6) étant formé d'une étoffe non-tissée (60) et ayant un côté fixe (62) le long d'un des bords latéraux de celui-ci et un côté libre (61) sur l'autre côté,
chaque revers anti-fuite (6) ayant des extrémités longitudinales opposées, l'une dans la portion avant (F) et l'autre dans la portion arrière (R), et ayant une portion d'extrémité fixe (65) fixée à la feuille supérieure (5) près de chaque extrémité longitudinale,

chaque revers anti-fuite (6) ayant une région élastique (64) dans laquelle un élément élastique (63) est fixé le long et près du bord libre (61),

le bord fixe (62) et la région élastique (64) de chaque revers anti-fuite (6) étant espacés latéralement à une première distance (DI) de 10 mm ou plus, et la région élastique (64) et la portion d'extrémité fixe (65) de chaque revers anti-fuite (6) étant espacées longitudinalement à une seconde distance (D2), la seconde distance (D2) étant égale ou inférieure à la première distance (D1) dans l'une au moins de la portion avant (F) et de la portion arrière (R), et

l'étoffe non-tissée (60) ayant un écart moyen de rugosité de surface (SMD) de 2,2 μm ou moins et un écart moyen de coefficient de frottement (MMD) de 0,012 ou moins, l'écart moyen de rugosité de surface et l'écart moyen de coefficient de frottement étant mesurés selon le procédé décrit dans la description,

dans lequel l'assemblage absorbant (2) comprend l'élément absorbant (4),

l'une au moins de la portion avant (F) et de la portion arrière (R) comprend une région de taille élastique à extension latérale (33) située plus proche de l'ouverture de taille (WH) que l'extrémité longitudinale de l'élément absorbant (4) et une paire de régions latérales élastiques à extension latérale (37) situées chacune latéralement à l'extérieur de l'assemblage absorbant (2),

chaque région latérale élastique (37) comprend une région d'extrémité fixe élastique (38), la région d'extrémité fixe élastique (38) contenant la portion d'extrémité fixe (65) et s'étendant latéralement vers l'extérieur depuis la portion d'extrémité fixe (65), et

la région d'extrémité fixe élastique (38) a une contrainte d'extension plus élevée que la région de taille élastique (33).

2. Article absorbant à enfiler selon la revendication 1, dans lequel la seconde distance (D2) est égale ou inférieure à la première distance (D1) dans les deux portions avant et arrière.

3. Article absorbant à enfiler selon la revendication 1 ou 2, dans lequel la portion d'extrémité fixe (65) a une première résistance au pelage de 0,2 à 5,0 N/20 mm entre l'étoffe non-tissée (60) et la feuille supérieure (5), et une région de jonction (66) incluant le bord fixe (62) a une résistance au pelage entre l'étoffe non-tissée (60) et un élément joint à celle-ci égale ou supérieure à la première résistance au pelage, la résistance au pelage étant mesurée selon le procédé décrit dans la description.

4. Article absorbant à enfiler selon la revendication 3, dans lequel la résistance au pelage de la région de jonction (66) incluant le bord fixe (62) est de 1,3 N/20 mm ou plus.

5. Article absorbant à enfiler selon la revendication 3 ou 4, dans lequel la région de jonction (66) incluant le bord fixe (62) a une dimension latérale plus petite que la portion d'extrémité fixe (65).

6. Article absorbant à enfiler selon l'une quelconque des revendications 1 à 5, dans lequel la première distance (D1) est de 15 mm ou plus.

7. Article absorbant à enfiler selon l'une quelconque des revendications 1 à 6, dans lequel la seconde distance (D2) est de 15 mm ou moins.

8. Article absorbant à enfiler selon l'une quelconque des revendications 1 à 7, dans lequel la portion d'extrémité fixe (65) a une pluralité de liaisons par fusion (81) où l'étoffe non-tissée (60) et la feuille supérieure (5) sont liées conjointement par fusion, et

le rapport de l'aire total des liaisons par fusion (81) sur l'aire de la portion d'extrémité fixe (65) est d'au moins 5 %.

9. Article absorbant à enfiler selon l'une quelconque des revendications 1 à 8, comprenant en outre une couverture extérieure (3) à laquelle l'assemblage absorbant (2) est fixé, la couverture extérieure (3) comprenant un panneau avant (3F) dans la portion avant (F) et un panneau arrière (3R) dans la portion arrière (R).

10. Article absorbant à enfiler selon l'une quelconque des revendications 1 à 9, dans lequel l'une au moins de la portion avant (F) et de la portion arrière (R) inclut : une région de taille élastique à extension latérale (33) située plus proche de l'ouverture de taille (WH) que l'extrémité de l'élément absorbant (4) et s'étendant le long d'une direction circonférentielle de la région de taille (33) ; et une paire de régions latérales élastiques à extension latérale (37) situées chacune latéralement à l'extérieur de l'assemblage absorbant (2),

chaque région latérale élastique (37) comprend une région d'extrémité fixe élastique (38) contenant la portion d'extrémité fixe (65) et s'étendant latéralement vers l'extérieur depuis la portion d'extrémité fixe (65),
la région d'extrémité fixe élastique (38) a une contrainte d'extension plus élevée que la région de taille élastique (33), la contrainte d'extension étant mesurée selon le procédé décrit dans la description.

11. Article absorbant à enfiler selon la revendication 10, dans lequel chaque région d'extrémité fixe élastique recouvre la portion d'extrémité fixe dans la portion d'extrémité latéralement proximale de celle-ci dans la direction de l'épaisseur.

12. Article absorbant à enfiler selon l'une quelconque des revendications 1 à 11, dans lequel chaque région d'extrémité fixe élastique (38) recouvre la portion d'extrémité fixe (65) dans la portion d'extrémité latéralement proximale de celle-ci dans la direction de l'épaisseur.

13. Article absorbant à enfiler selon l'une quelconque des revendications 1 à 12, dans lequel l'assemblage absorbant (2) comprend un élément absorbant (4), et
l'élément absorbant (4) a une fente (44) ou une région à faible poids de base dans les portions latérales opposées de celui-ci, la fente (44) ou la région à faible poids de base s'étendant longitudinalement en parallèle à une région de jonction (66) incluant le bord fixe (62).

14. Article absorbant à enfiler selon la revendication 13, dans lequel la portion d'extrémité fixe (65) est située latéralement à l'extérieur de la fente (44) ou de la région à faible poids de base.

Fig. 1

Fig. 2

EP 3 949 922 B1

Fig. 3

Fig. 4

Fig. 5

Fig. 6

18

Fig. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017023782 A
- JP 2019010348 A

- WO 2019038956 A1

**Non-patent literature cited in the description**

- Japanese Hand Evaluation and Standardization Committee (HESC). **KAWABATA, HIDEO**. fuai hyouka no hyoujunka to kaiseki (Standardization and Analysis of Hand Evaluation). The Textile Machinery Soc. of Japan, 10 July 1980